# EUROPÄISCHE PATENTSCHRIFT

(11) **EP 2 171 441 B1**
(45) Veröffentlichungstag und Bekanntmachung des Hinweises auf die Patenterteilung: **17.12.2014**
(21) Anmeldenummer: 08774695.4
(22) Anmeldetag: 03.07.2008
(51) Int. Cl.: G01N 27/414

(54) **Anordnung von Gassensoren**
Arrangement of gas sensors
Ensemble de capteurs de gaz

(30) Priorität: 24.07.2007 DE 102007034331
(43) Veröffentlichungstag der Anmeldung: 07.04.2010
(73) Patentinhaber: Robert Bosch GmbH, 70442 Stuttgart (DE)
(72) Erfinder: WOLST, Oliver, 72622 Nuertingen (DE); LE-HUU, Martin, 70825 Korntal (DE)
(86) Internationale Anmeldenummer: PCT/EP2008/058574
(87) Internationale Veröffentlichungsnummer: WO 2009/013103

(56) Entgegenhaltungen:
- DE-A1- 10 331 299
- FR-A- 2 830 327
- US-A1- 2005 170 347
- US-B2- 6 575 013

## Beschreibung

### Stand der Technik

Die Erfindung geht aus von einer Vorrichtung zur Detektierung von in einem Fluidstrom enthaltenen Substanzen gemäß dem Oberbegriff des Anspruchs 1. Weiterhin betrifft die Erfindung ein Verfahren zur Detektierung von Substanzen in einem Fluidstrom, bei dem mehrere als Messsensoren wirkende Feldeffekttransistoren parallel geschaltet sind.

Gassensitive Feldeffekttransistoren auf Halbleiterbasis werden in der Gassensorik als Messsensoren eingesetzt. Üblicherweise führt dabei die Beaufschlagung mit einem zu detektierenden Gas zu einer Veränderung des von der Source-Elektrode zur Drain-Elektrode durch den Transistor fließenden Stroms, dem so genannten Kanalstrom.

Zur Detektierung von mehreren unterschiedlichen Gasen ist es zum Beispiel aus US-B 6,575,013 bekannt, mehrere Messsensoren in einer Anordnung zu verschalten. Die Messsensoren sprechen dabei jeweils auf unterschiedliche Gase an. Um die Signale auswerten zu können, führt von jedem Messsensor eine Signalleitung zu einer Auswerteeinheit. Nachteil der beschriebenen Vorrichtung ist jedoch, dass bei einer erforderlichen räumlichen Trennung der Detektion und der Auswertung, wie sie zum Beispiel bei der Detektion heißer Gase erforderlich ist, eine erhebliche Anzahl an Signalleitungen erforderlich ist. Wenn in einem Sensormodul somit die Auswertung mehrerer Messsensoren vorgesehen ist, kann die Anzahl an Signalleitungen erheblich zu den Modulkosten beitragen. Es ist somit sinnvoll, Maßnahmen zur Reduzierung der Anzahl der erforderlichen Signalleitungen zu ergreifen.

Eine weitere Möglichkeit, mehrere Signale von verschiedenen Sensoren auszulesen, ist die Verwendung von Multiplexern. Diese verfügen über mehrere Eingänge und einen Ausgang und verschalten in Abhängigkeit eines Steuersignals die an einem Eingang anliegende Spannung mit dem Ausgang. Bei gassensitiven Feldeffekttransistoren ist es jedoch üblich, den Strom als Messgröße zu nutzen. Jedoch ist das Multiplexen von Strom sehr aufwändig und mit den hohen Anforderungen an Temperatur- und Signalstabilität, die an gassensitive Feldeffekttransistoren gestellt werden, nicht vereinbar.

Aus der DE 103 312 99 A1 ist ein Sensor-Transistor-Element, eine Sensor-Einheit und ein Sensor-Array bekannt. Auf der Gate- isolierenden Schicht und über der Gate-Elektrode ist eine Kanal-Schicht zwischen der ersten Source-/Drain-Elektrode und der zweiten Source-/Drain-Elektrode gebildet, welche Kanalschicht aus Fängermolekülen gebildet ist, die derart eingerichtet sind, dass sie mit in einem Analyten möglicherweise enthaltenden zu erfassenden Partikeln hybridisieren und so die Leitfähigkeit im Kanal modifizieren.

### Offenbarung der Erfindung

### Vorteile der Erfindung

Eine erfindungsgemäß ausgebildete Vorrichtung zur Detektierung von in einem Fluidstrom enthaltenen Substanzen umfasst mindestens zwei Feldeffekttransistoren als Messsensoren, wobei die Feldeffekttransistoren jeweils eine Gate-Elektrode, eine Source-Elektrode und eine Drain-Elektrode aufweisen. Die Source-Elektroden und die Drain-Elektroden aller als Messsensoren wirkenden Feldeffekttransistoren sind parallel geschaltet, zwischen dem gemeinsamen Drain- und dem gemeinsamen Source-Anschluss liegt eine Betriebsspannung an. Die Vorrichtung umfasst weiterhin Mittel zum Schalten, wobei Steuerspannungen an den Eingängen der Mittel zum Schalten angelegt sind und die Gate-Elektrode jedes als Messsensor wirkenden Feldeffekttransistors mit einem Ausgang eines Mittels zum Schalten verbunden ist, so dass an alle als Messsensor wirkenden Feldeffekttransistoren unabhängig voneinander eine der Steuerspannungen angelegt werden kann. Eine der Steuerspannungen liegt dabei auf einem Spannungsniveau, das zu einer Verarmung der Ladungsträger im Halbleiterkanal zwischen Source und Drain führt (Sperrspannung). Eine weitere Steuerspannung definiert einen Arbeitspunkt der Feldeffekttransistoren. Weitere Steuerspannungen können dazu dienen, die als Messsensor wirkenden Feldeffekttransistoren in anderen Arbeitspunkten zu betreiben.

Vorteil der Verwendung von Feldeffekttransistoren als Messsensoren ist, dass diese nur eine kleine Baugröße aufweisen und in hochparallelen Halbleiterprozessen zu geringen Kosten hergestellt werden können. Durch die Verwendung von mindestens zwei als Messsensor wirkenden Feldeffekttransistoren ist eine redundante Auslegung, d. h. der Einsatz mehrerer baugleicher Feldeffekttransistoren zur Erhöhung der Ausfallsicherheit, als auch eine Kombination von als Messsensor wirkenden Feldeffekttransistoren mit unterschiedlichen Sensitivitäten zur Detektion mehrerer Testgase beziehungsweise zur Kontrolle von Querempfindlichkeiten, d. h. das Ansprechen eines als Messsensor wirkenden Feldeffekttransistors auf mehrere Testgase, möglich.

Durch die Verwendung der Mittel zum Schalten ist es möglich, dass jeweils nur an einen als Messsensor wirkenden Feldeffekttransistor eine Gatespannung im Arbeitspunkt angelegt wird, während an alle anderen als Messsensor wirkenden Feldeffekttransistoren die Sperrspannung angelegt wird. Dies führt dazu, dass nur der als Messsensor wirkende Feldeffekttransistor, an den die Gatespannung im Arbeitspunkt angelegt ist, ein Signal (Kanalstrom) liefert. Auf diese Weise ist es möglich, mehrere als Messsensor wirkende Feldeffekttransistoren der Vorrichtung an eine gemeinsame Signalleitung anzuschließen. Besonders bevorzugt werden alle als Messsensor wirkenden Feldeffekttransistoren der Vorrichtung an eine gemeinsame Signalleitung angeschlossen.

Die Mittel zum Schalten, mit denen der als Messsensor wirkende Feldeffekttransistor entweder mit der Gatespannung im Arbeitspunkt oder der Sperrspannung versorgt wird, können entweder in Form einer integrierten Schaltung zusammen mit dem als Messsensor wirkenden Feldeffekttransistor auf einem Chip ausgebildet sein, oder es werden zwei getrennte Chips eingesetzt, wobei einer die Mittel zum Schalten in Form eines Schaltungsmoduls und der andere die als Messsensor wirkenden Feldeffekttransistoren trägt.

Ein weiterer Vorteil des Einsatzes von wenigen oder nur einer Signalleitung ist, dass gegebenenfalls auftretende Probleme in der Kontaktierung der Signalleitungen an den Messsensor minimiert werden.

Die Vorrichtung umfasst in einer bevorzugten Ausführungsform weiterhin eine digitale Schaltung, die das Anlegen der Gatespannung im Arbeitspunkt oder der Sperrspannung an die als Messsensor wirkenden Feldeffekttransistoren steuert. In einer möglichen Ausführung umfasst die digitale Schaltung einen Taktgenerator, einen sequenziellen Zähler und eine bool'sche Logik. Die bool'sche Logik umfasst dabei vorzugsweise auch die Mittel zum Schalten. Durch die digitale Schaltung werden so sämtliche Gate-Elektroden der als Messsensoren wirkenden Feldeffekttransistoren der Vorrichtung angesteuert, um jeweils einen als Messsensor wirkenden Feldeffekttransistor derart zu schalten, dass dieser ein Messsignal abgibt, und alle anderen als Messsensoren wirkenden Feldeffekttransistoren zu sperren. Mit dem Taktgenerator wird ein Durchschalten der als Messsensoren wirkenden Feldeffekttransistoren ermöglicht, indem zum Beispiel sequenziell mit Hilfe der bool'schen Logik und der Mittel zum Schalten ein als Messsensor wirkender Feldeffekttransistor nach dem anderen aktiviert und wieder deaktiviert wird. Der sequenzielle Zähler zählt von 0 bis zur Anzahl der in der Vorrichtung enthaltenen als Messsensoren wirkenden Feldeffekttransistoren. Dies erfolgt üblicherweise binär mit der erforderlichen Anzahl von Bits. Die bool'sche Logik wertet den Zählerstand für jeden Sensor aus. Ergibt die Auswertung eine logische "1" schaltet das Mittel zum Schalten für den entsprechenden Sensor die Gatespannung auf die Spannung im Arbeitspunkt. Ergibt die Auswertung eine logische "0" schaltet das Mittel zum Schalten für den entsprechenden Sensor die Gatespannung auf die Sperrspannung. Bei einer exklusiven Zuordnung der Zählerstandes zu nur jeweils einem als Messsensor wirkenden Feldeffekttransistor, ergibt die Auswertung durch die bool'sche Logik für einen Zählerstand nur für den zugeordneten Messsensor eine logische "1", für alle anderen Messsensoren eine logische "0". Es wird sequenziell über alle als Messsensoren wirkenden Feldeffekttransistoren gezählt, wobei jeder als Messsensor wirkender Feldeffekttransistor ein Signal abgibt. Sobald der letzte Sensor erreicht ist, wird das Zählersignal wieder auf 0 gesetzt. Hierzu werden alle als Messsensoren wirkenden Feldeffekttransistoren gesperrt. Diese Phase wird als Synchronisationsphase bezeichnet. Anschließend beginnt der Auslesezyklus wieder beim ersten Sensor. So ist eine Zuordnung des Signals zum entsprechenden Sensor möglich.

Um Störgrößen zu eliminieren, sind in einer bevorzugten Ausführungsform die als Messsensor wirkenden Feldeffekttransistoren mit einem Referenzsensor verbunden. Der Referenzsensor ist vorzugsweise ein weiterer Feldeffekttransistor, der in seinem Aufbau mit dem der als Messsensoren wirkenden Feldeffekttransistoren übereinstimmt. Der Referenzsensor unterscheidet sich jedoch von den als Messsensoren wirkenden Feldeffekttransistoren dadurch, dass dieser gegenüber den zu detektierenden Substanzen unempfindlich ist. Dies wird zum Beispiel dadurch erzielt, dass die Gate-Elektrode des als Referenzsensor eingesetzten Feldeffekttransistors passiviert wird. Dies kann zum Beispiel durch dem Fachmann bekannte Verfahren zur Abscheidung von Passivierungsmaterial auf der Gate-Elektrode erfolgen. Das Abscheiden erfolgt zum Beispiel durch Aufdampfen oder Aufsputtern. Als Passivierungsmaterial eignen sich zum Beispiel Keramiken oder organische Polymere, die für die zu erfassenden Substanzen dicht sind.

In einer ersten Ausführungsform wird der Referenzsensor mit allen parallel geschalteten, als Messsensor wirkenden Feldeffekttransistoren in Reihe geschaltet. Vorteil dieser Verschaltung ist es, dass ein einziger Referenzsensor ausreicht, um die Störsignale zu eliminieren.

In einer alternativen Ausführungsform werden ein oder mehrere Referenzsensoren zu allen parallel geschalteten, als Messsensor wirkenden Feldeffekttransistoren parallel geschaltet. Dabei sind die Referenzsensoren weitere Sensoren im Aufnahmezyklus und werden genauso wie die als Messsensoren wirkenden Feldeffekttransistoren einmal ausgelesen. Hierbei ist es erforderlich, dass die Dauer des Auslesezyklusses klein ist gegen typische Zeitskalen der zu eliminierenden Störeinflüsse. Das Messsignal der Referenzsensoren wird mit dem der als Messsensoren wirkenden Feldeffekttransistoren verglichen.

Hieraus lassen sich ebenfalls die Störgrößen eliminieren. Nachteil des parallel geschalteten Referenzsensors gegenüber dem in Reihe geschalteten Referenzsensor ist, dass das Signal an die Auswerteeinheit übergeben werden muss und eine Eliminierung der Störsignale erst in der Auswerteeinheit erfolgt. Bei dem in Reihe geschalteten Referenzsensor werden die Störsignale direkt in der Schaltung eliminiert.

In noch einer weiteren Ausführungsform wird zu jedem als Messsensor wirkenden Feldeffekttransistor ein Referenzsensor parallel geschaltet. Vorteil dieser Ausführungsform ist es, dass spezifisch für jeden als Messsensor wirkenden Feldeffekttransistor ein spezieller Referenzsensor eingesetzt werden kann. Dieser kann an die Eigenschaften des jeweiligen als Messsensor wirkenden Feldeffekttransistors angepasst werden.

In einer weiteren Ausführungsform erfolgt die Selektion der auszulesenden als Messsensoren wirkenden Feldeffekttransistoren nicht über das Anlegen einer Steuerspannung, sondern über einen zu jedem als Messsensor wirkenden Feldeffekttransistor in Reihe geschalteten Schalt-Feldeffekttransistor. Dieser Schalt-Feldeffekttransistor wird nun wiederum mit einer entsprechenden Steuerspannung entweder gesperrt (Sperrspannung) oder in einen Arbeitspunkt geschaltet. Eine solche Anordnung vermeidet es, das elektro-chemische Gleichgewicht des chemisch sensitiven Gates der als Messsensor wirkenden Feldeffekttransistoren durch den Schaltvorgang zu beeinflussen, da an die Gate-Elektroden aller als Messsensoren wirkenden Feldeffekttransistoren eine konstante Gatespannung im Arbeitspunkt angelegt werden kann. Diese wird durch den Schaltvorgang nicht verändert. Zudem erlaubt es durch die Trennung von Schaltung und Fluid-Detektion eine Optimierung der verwendeten Feldeffekttransistoren auf ihre jeweilige Aufgabe. Insbesondere sind bei den als Messsensoren wirkenden Feldeffekttransistoren aufgrund der elektro-chemischen Anforderungen an die Gategeometrie höhere Leckströme und ein schlechteres Abschnürverhalten zu erwarten als bei einem zur Schaltung optimierten Schalt-Feldeffekttransistor.

Die Erfindung betrifft weiterhin ein Verfahren zur Detektierung von Substanzen in einem Fluidstrom, bei dem mehrere als Messsensoren wirkende Feldeffekttransistoren parallel geschaltet sind und jeweils an einen als Messsensor wirkenden Feldeffekttransistor die Gatespannung im Arbeitspunkt und an alle anderen als Messsensor wirkenden Feldeffekttransistoren die Sperrspannung angelegt ist, so dass jeweils nur ein als Messsensor wirkender Feldeffekttransistor ein Messsignal liefert. Die Selektion der Signale der als Messsensor wirkenden Feldeffekttransistoren erfolgt durch das gezielte Abschnüren der Kanäle durch das Anlegen eines entsprechenden Gate-Potenzials mit Hilfe zum Beispiel einer auf dem Sensorchip realisierten integrierten Schaltung. Auf diese Weise können die Signale aller als Messsensor wirkenden Feldeffekttransistoren unterdrückt werden. Dies führt dazu, dass kein Strom durch die Anordnung fließt. Sobald nun das Gate-Potenzial eines als Messsensor wirkenden Feldeffekttransistors auf seinen Arbeitspunkt gelegt wird, fließt Strom nur durch diesen einen als Messsensor wirkenden Feldeffekttransistor. Hierdurch gelingt es, den Strom eines einzelnen als Messsensor wirkenden Feldeffekttransistors auszulesen, ohne diesen durch eine nachfolgende Schaltung zum Beispiel durch Serienwiderstände oder parasitäre Ströme zu verändern. Wenn das Umschalten zwischen den einzelnen als Messsensor wirkenden Feldeffekttransistoren innerhalb deren Schaltfrequenz, die im Allgemeinen im Bereich einiger kHz liegt, erfolgt, ist auch der Einfluss unvermeidbarer Einschwingvorgänge auf das Stromsignal zu vernachlässigen. Im Unterschied zu einem Strommultiplexer ist dieses Verfahren ähnlich einfach wie ein Spannungsmultiplexer zu realisieren und problemlos für eine Anzahl von als Messsensor wirkenden Feldeffekttransistoren der erfindungsgemäßen Vorrichtung skalierbar.

In einer bevorzugten Ausführungsform wird die Gate-Spannung im Arbeitspunkt sequenziell an einen als Messsensor wirkenden Feldeffekttransistor nach dem anderen angelegt. Hierdurch ist eine sequenzielle Auslesung der als Messsensor wirkenden Feldeffekttransistoren und damit eine genaue Zuordnung der Signale zu den einzelnen als Messsensor wirkenden Feldeffekttransistoren möglich. Dies ist insbesondere dann sinnvoll, wenn mit den einzelnen als Messsensor wirkenden Feldeffekttransistoren jeweils unterschiedliche Substanzen detektiert werden können.

In einer bevorzugten Ausführungsform wird zur Eliminierung von Störgrößen ein Messwert aus dem Messsignal eines als Messsensor wirkenden Feldeffekttransistors und einem Referenzsignal eines Referenzsensors gebildet. Hierdurch wird ein Signaloffset, der unter anderem von Störeinflüssen wie Temperaturschwankungen, Gate-Potenzial-Schwankungen oder Prozessabweichungen abhängig ist, kompensiert. Der Referenzsensor ist dabei wie oben beschrieben derart ausgebildet, dass dieser nicht von den zu detektierenden Substanzen beeinflusst wird.

### Kurze Beschreibung der Zeichnungen

Ausführungsformen der Erfindung sind in den Zeichnungen dargestellt und werden in der nachfolgenden Beschreibung näher erläutert.

Es zeigen:
Figur 1 ein Schaltbild einer erfindungsgemäß ausgebildeten Vorrichtung,
Figur 2 exemplarisch einen Signalverlauf der in Figur 1 dargestellten Schaltung,
Figur 3 ein Schaltbild der erfindungsgemäßen Vorrichtung mit digitaler Schaltung,
Figur 4 ein Schaltbild einer erfindungsgemäß ausgebildeten Vorrichtung mit einem in Reihe geschalteten Referenzsensor,
Figur 5 ein Schaltbild einer erfindungsgemäß ausgebildeten Vorrichtung mit parallel geschalteten Referenzsensoren,
Figur 6 ein Schaltbild einer erfindungsgemäß ausgebildeten Vorrichtung mit Feldeffekttransistoren als Mittel zum Schalten.

### Ausführungsformen der Erfindung

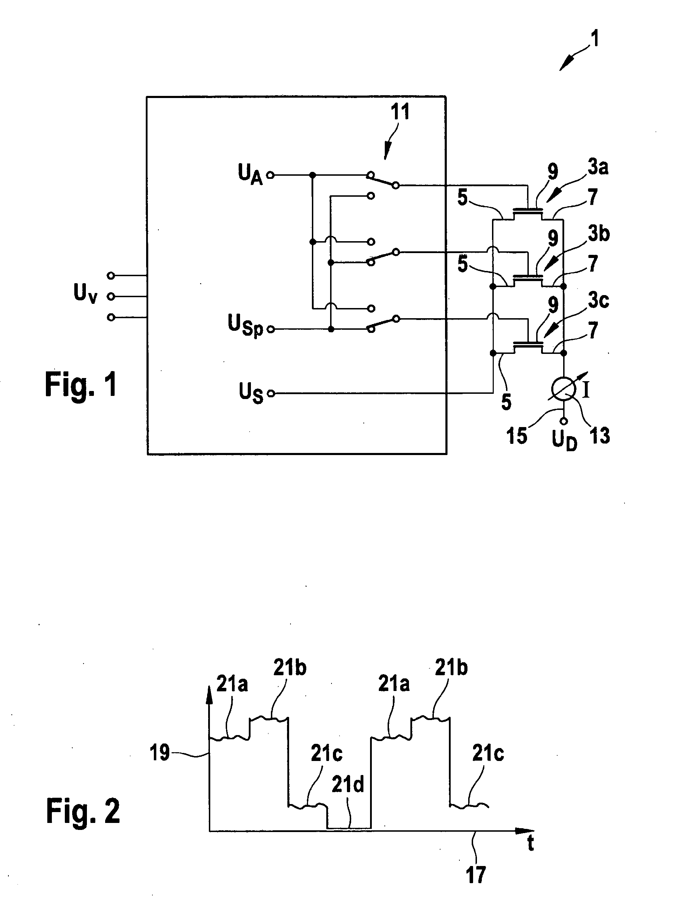

In Figur 1 ist ein Schaltbild einer erfindungsgemäß ausgebildeten Vorrichtung dargestellt. Bei einer erfindungsgemäß ausgebildeten Vorrichtung 1 sind mehrere als Messsensor wirkende Feldeffekttransistoren 3a, 3b, 3c parallel geschaltet. Hierzu sind die Source-Elektroden 5 der als Messsensor wirkenden Feldeffekttransistoren 3a, 3b, 3c mit einer gemeinsamen Source-Spannung U_{S} und die Drain-Elektroden 7 der als Messsensor wirkenden Feldeffekttransistoren 3a, 3b, 3c mit einer Drain-Spannung U_{D} verbunden. Die Differenz zwischen der Source-Spannung U_{S} und der Drain-Spannung U_{D} ist die Betriebsspannung der als Messsensor wirkenden Feldeffekttransistoren 3a, 3b, 3c. Auf diese Weise erfolgt eine Parallelverschaltung von Source-Elektrode 5 und Drain-Elektrode 7 aller als Messsensoren eingesetzten Feldeffekttransistoren 3a, 3b, 3c. Die Anzahl der eingesetzten als Messsensor wirkenden Feldeffekttransistoren 3a, 3b, 3c, die parallel verschaltet werden, ist im Allgemeinen kleiner als 10. Es ist jedoch auch möglich, mehr als 10 als Messsensor wirkende Feldeffekttransistoren parallel zu schalten.

Die Messgröße der Vorrichtung 1 zur Detektierung von in einem Fluidstrom enthaltenen Substanzen ist der durch die Anordnung der als Messsensor wirkenden Feldeffekttransistoren 3a, 3b, 3c fließende Strom zwischen der Gesamt-Source und. Gesamt-Drain. Der Strom ist üblicherweise kleiner als 10⁻¹ A. Der Strom kann dabei wahlweise als Funktion der angelegten Spannung oder bei einer konstanten Spannung gemessen werden. Die Spannung ist typischerweise kleiner als +/- 20 V. Durch das Anlegen einer Spannung zwischen der Source-Elektrode 5 und der Gate-Eiektrode 9 kann der Kanalstrom eines als Messsensor wirkenden Feldeffekttransistors 3a, 3b, 3c verändert werden. Die Spannung zwischen der Source-Elektrode 5 und der Gate-Elektrode 9 ist die so genannte Gate-Spannung oder Steuerspannung. Relevante Gate-Spannungen sind die Sperrspannung, die im Allgemeinen kleiner als +/-10 V ist, bei der durch eine Abschnürung des Transistorkanals der Kanalstrom unterdrückt wird. Weiterhin relevant ist eine frei wählbare Gate-Spannung im Arbeitspunkt, der ebenfalls bei einer Spannung liegt, die im Allgemeinen kleiner als +/- 10 V ist. Bei dem Arbeitspunkt wird der Einfluss der Substanz auf den Kanalstrom gemessen.

Erfindungsgemäß wird nur die Gate-Spannung eines als Messsensor wirkenden Feldeffekttransistors 3a, 3b, 3c zu einem Zeitpunkt auf den Arbeitspunkt gelegt. Alle anderen als Messsensor wirkenden Feldeffekttransistoren werden durch Anlegen der Sperrspannung gesperrt. Der Strom zwischen dem Gesamt-Source und dem Gesamt-Drain der Anordnung der als Messsensor wirkenden Feldeffekttransistoren 3a, 3b, 3c hängt somit nur vom Kanalstrom des geöffneten als Messsensor wirkenden Feldeffekttransistors 3a, 3b, 3c ab.

Um die Gate-Spannung jeweils nur eines als Messsensor wirkenden Feldeffekttransistors 3a, 3b, 3c auf den Arbeitspunkt zu legen, sind die Gate-Elektroden 9 der als Messsensor wirkenden Feldeffekttransistoren 3a, 3b, 3c jeweils mit einem Ausgang eines Mittels zum Schalten 11 verbunden. An die Eingänge der Mittel zum Schalten 11 sind zum einen die Gate-Spannung im Arbeitspunkt U_{A} und zum anderen die Sperrspannung U_{Sp} angelegt. Durch Schalten der Mittel zum Schalten 11 lässt sich so auf einfache Weise an die Gate-Elektrode 9 entweder die Gate-Spannung im Arbeitspunkt U_{A} oder die Sperrspannung U_{Sp} anlegen.

Um die Vorrichtung 1 betreiben zu können, ist an diese eine Versorgungsspannung U_{V} angelegt.

Um die in dem Fluidstrom enthaltenen Substanzen zu detektieren, wird nun zunächst durch Schalten der Mittel zum Schalten 11 an die Gate-Elektrode 9 eines der als Messsensor wirkenden Feldeffekttransistoren 3a, 3b, 3c die Gate-Spannung im Arbeitspunkt U_{A} angelegt und an alle anderen die Sperrspannung U_{Sp}. In der in Figur 1 dargestellten Verschaltung ist an den obersten als Messsensor wirkenden Feldeffekttransistor 3a die Gate-Spannung im Arbeitspunkt U_{A} und an die anderen beiden als Messsensor wirkenden Feldeffekttransistoren 3b, 3c die Sperrspannung U_{Sp} angelegt. Im weiteren Messverlauf wird dann der oberste als Messsensor wirkende Feldeffekttransistor 3a durch Schalten des Mittels zum Schalten 11 von der Gate-Spannung im Arbeitspunkt U_{A} getrennt und mit der Sperrspannung U_{Sp} versorgt. Gleichzeitig wird einer der beiden als Messsensor wirkenden Feldeffekttransistoren 3b, 3c, die mit der Sperrspannung U_{Sp} versorgt werden, durch Schalten des Mittels zum Schalten 11 von der Sperrspannung U_{Sp} getrennt und mit der Gate-Spannung im Arbeitspunkt U_{A} versorgt. Auf diese Weise wird der Reihe nach jeweils ein als Messsensor wirkender Feldeffekttransistor 3 mit der Gate-Spannung im Arbeitspunkt U_{A} versorgt und kann so zur Messung eingesetzt werden.

Das Messsignal ist der auf der Drain-Seite zu messende Strom, die Messung wird durch das dargestellte Messgerät 13 illustriert.

Erfindungsgemäß ist an einen als Messsensor wirkenden Feldeffekttransistor 3a, 3b, 3c sequenziell die Gate-Spannung im Arbeitspunkt U_{A} angelegt, während an den anderen als Messsensor wirkenden Feldeffekttransistoren 3a, 3b, 3c die Sperrspannung U_{Sp} anliegt. Auf diese Weise können die als Messsensor wirkenden Feldeffekttransistoren 3a, 3b, 3c nacheinander über eine gemeinsame Signalleitung 15 ausgelesen werden. Ein derartiges Messsignal ist beispielhaft in Figur 2 dargestellt. Dabei ist auf der x-Achse 17 die Zeit t und auf der y-Achse 19 das aufgenommene Signal dargestellt. Durch sequenzielles Anlegen der Gate-Spannung im Arbeitspunkt U_{A} wird zunächst ein erstes Signal 21a des ersten als Messsensor wirkenden Feldeffekttransistors 3a, dann ein zweites Signal 21b des zweiten als Messsensor wirkenden Feldeffekttransistors 3b und ein drittes Signal des dritten als Messsensor wirkenden Feldeffekttransistors 3c aufgenommen. Die Aufnahme der Signale 21a, 21b und 21c erfolgt jeweils durch Schalten des entsprechenden Mittels zum Schalten 11 von der Sperrspannung U_{Sp} auf die Gate-Spannung im Arbeitspunkt U_{A} und endet mit dem Schalten des Mittels zum Schalten 11 von der Gate-Spannung im Arbeitspunkt U_{A} auf die Sperrspannung U_{Sp}. Da die einzelnen als Messsensor wirkenden Feldeffekttransistoren 3a, 3b und 3c unterschiedliche Substanzen detektieren sollen, liefern diese auch unterschiedliche Signale 21a, 21b und 21c. Aus dem Signal lässt sich erkennen, ob die von dem jeweiligen als Messsensor wirkenden Feldeffekttransistor 3a, 3b oder 3c zu detektierende Substanz im Fluidstrom enthalten ist und in welcher Menge. Weiterhin ist ein Schaltzustand 21d zu erkennen, in dem an allen als Messsensor wirkenden Feldeffekttransistoren 3a, 3b, 3c die Sperrspannung U_{SP} anliegt und somit kein Strom fließt. Dieser Zustand dient der Synchronisation mit der externen Auswerteschaltung.

Der zu detektierende Fluidstrom ist erfindungsgemäß ein Gasstrom, und die zu detektierende Substanz liegt im Allgemeinen ebenfalls gasförmig vor.

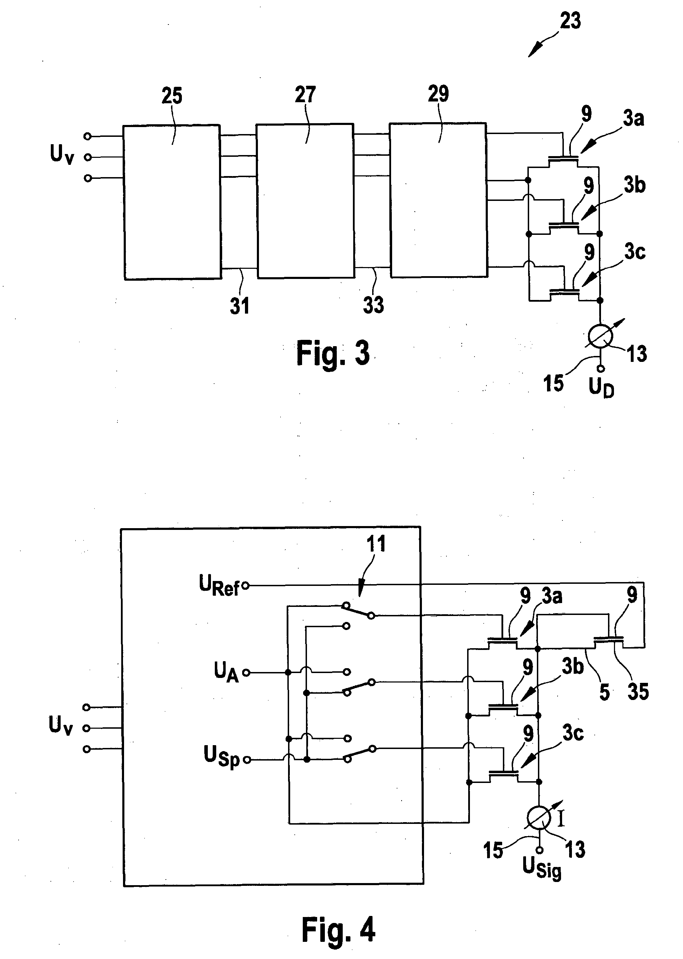

Um die einzelnen als Messsensor wirkenden Feldeffekttransistoren 3a, 3b und 3c getrennt ansteuern zu können, ist eine digitale Schaltung 23 vorgesehen, wie sie in Figur 3 dargestellt ist.

Die digitale Schaltung 23 umfasst in der hier dargestellten Ausführungsform drei Komponenten. Eine Komponente ist ein Taktgenerator 25, eine zweite ein sequenzieller Zähler 27 und die dritte eine bool'sche Logik 29. Weiterhin umfasst die digitale Schaltung 23 die Mittel zum Schalten 11.

Mit dem Taktgenerator 25 wird die Messdauer für jeden einzelnen als Messsensor wirkenden Feldeffekttransistor 3a, 3b und 3c festgelegt. Die Messdauer liegt im Allgemeinen im Bereich von 0.1 bis 10000 ms. Anstelle eines integrierten Taktgenerators 25 ist es auch möglich, das Taktsignal extern anzulegen. Das vom Taktgenerator 25 ausgehende Taktsignal 31 wird dem sequenziellen Zähler 27 zugeführt. Der sequenzielle Zähler 27 zählt jeweils von 0 bis zur Anzahl der in der Vorrichtung 1 verschalteten als Messsensor wirkenden Feldeffekttransistoren 3a, 3b, 3c. Dies erfolgt im Allgemeinen binär durch die erforderliche Anzahl von Bits. Sobald nun der Taktgenerator 25 ein Taktsignal 31 an den sequenziellen Zähler 27 liefert, zählt dieser um 1 weiter und liefert ein Zählersignal 33 an die bool'sche Logik 29.

Die bool'sche Logik wertet den Zählerstand für jeden als Messsensor wirkenden Feldeffektransistor 3a, 3b und 3c aus. Mit der bool'schen Logik 29 werden die einzelnen Mittel zum Schalten 11 gesteuert. Die logische "1" kann hierbei zum Beispiel das Mittel zum Schalten auf die Gate-Spannung im Arbeitspunkt U_{A} der als Messsensor wirkenden Feldeffekttransistoren 3a, 3b und 3c legen, die logische "0" auf die Sperrspannung U_{S}. Ergibt die Auswertung des Zählersignals 33 durch die bool'sche Logik für einen der als Messsensor wirkenden Feldeffekttransistoren 3a, 3b oder 3c eine logische "1", schaltet das entsprechende Mittel zum Schalten also auf die Gate-Spannung im Arbeitspunkt U_{A} für diesen als Messsensor wirkenden Feldeffektransistor 3a, 3b, 3c und legt diese an die Gate-Elektrode 9 an. Die Auswertung dieses Zählerstandes durch die bool'sche Logik ergibt für die anderen als Messsensor wirkenden Feldeffekttransistoren 3a, 3b, 3c eine logische "0" so dass die Mittel zum Schalten weiterhin die Sperrspannung U_{Sp} an die Gates 9 der jeweiligen als Messsensor wirkenden Feldeffekttransistoren 3a, 3b, 3c anlegen. Neben dem beschriebenen sequenziellen Schalten der als Messsensor wirkenden Feldeffekttransistoren 3a, 3b, 3c sind weitere Schaltzustände denkbar, die jeweils einem weiteren Zählerstand zugeordnet werden. Dies kann zum einen das gleichzeitige Schalten von mehreren als Messsensoren wirkenden Feldeffekttransistoren 3a, 3b, 3c in den Arbeitspunkt sein, um eine Substanz mit Hilfe eines Summensignals zu detektieren. Zum anderen ist es vorteilhaft, einen Schaltzustand zu realisieren, der von der externen Auswerteschaltung eindeutig als Synchronisationspuls erkannt wird. Dieser Zustand sollte beispielsweise durch einen besonders geringen oder besonders hohen Strom gekennzeichnet sein, der außerhalb des Signalbereichs liegt. Dies ermöglicht mindestens einmal während des Schaltzyklus eine eindeutige Bestimmung des Schaltzustands. Schaltungszustände, die von der externen Auswerteschaltung eindeutig als Synchronisationspuls erkannt werden können, sind zum Beispiel das Sperren aller als Messsensor wirkenden Feldeffekttransistoren oder das Öffnen aller als Messsensor wirkenden Feldeffekttransistoren.

Wenn entweder die Gesamt-Source oder Gesamt-Drain, d.h. einer der Kanalanschlüsse der als Messsensor wirkenden Feldeffekttransistoren 3a, 3b, 3c, an eine Versorgungsspannung U_{V} der digitalen Schaltung 23 angeschlossen wird, kann das Signal der Anordnung der als Messsensor wirkenden Feldeffekttransistoren 3a, 3b, 3c als Strom am anderen Kanalanschluss durch eine einzige Signalleitung 15 ausgelesen werden. In der hier dargestellten Anordnung ist die Source-Elektrode 5 an eine Spannungsversorgung U_{V} der digitalen Schaltung 23 angelegt, und die Drain-Elektroden 7 sind an die Drain-Spannung U_{D}, die gleichzeitig die Signalspannung darstellt, angelegt. Alternativ ist es jedoch auch möglich, dass die Drain-Elektroden 7 an die Versorgungsspannung U_{V} angelegt werden und die Signalleitung 15 an die Source-Elektroden 5.

Zusätzlich werden weitere Versorgungsanschlüsse der digitalen Schaltung 23 und gegebenenfalls ein Eingang für einen externen Takt benötigt. Die Anzahl der Versorgungsanschlüsse der digitalen Schaltung 23 liegt üblicherweise bei 2 oder 3.

Da der Kanalstrom eines als Messsensor wirkenden Feldeffekttransistors 3a, 3b, 3c stark von der Gate-Spannung, d.h. der Spannung zwischen Source-Elektrode 5 und Gate-Elektrode 9 abhängt, stellt die stabile Ansteuerung der Gate-Elektroden 9 durch die digitale Schaltung 23 eine Herausforderung dar. Eine Lösung besteht darin, für die Gate-Spannung im Arbeitspunkt eine der Versorgungspannungen U_{V} zu wählen. Für selbstleitende Feldeffekttransistoren kann die Gate-Spannung im Arbeitspunkt identisch sein mit der Source-Spannung U_{S}, im Allgemeinen kann eine geeignete von U_{S} verschiedene Versorgungsspannung gewählt werden. Das Messsignal ist insbesondere dann stabil, wenn der als Messsensor wirkende Feldeffekttransistor 3a, 3b, 3c im Sättigungsbereich betrieben wird. Im Sättigungsbereich zeigt der Kanalstrom nämlich nur eine geringe oder keine Abhängigkeit von der Drain-Source-Spannung. Dies führt dazu, dass das Signal dann unempfindlich gegenüber Schwankungen in der Versorgungsspannung ist.

Die Logik dient in der hier dargestellten Ausführungsform nicht zur Generierung des Gate-Potenzials, sondern entscheidet nur bezüglich des Schaltzustandes der Mittel zum Schalten 11. Solange die Störeinflüsse also klein sind gegen den Potenzialunterschied, der notwendig ist, um die Mittel zum Schalten 11 zu schalten, haben Störeinflüsse auf die Logik keinen Einfluss auf den Schaltzustand der Mittel zum Schalten 11. Die Gate-Spannungen sind durch die Steuerspannungen U_{A} und U_{SP} definiert, die identisch sind mit geeigneten Versorgungspannungen U_{V}.

Weiterhin ist es möglich, dass der logische Spannungslevel "0" identisch ist mit der Sperrspannung und die logische "1" identisch ist mit der Gate-Spannung im Arbeitspunkt. Dadurch kann auf die Mittel zum Schalten verzichtet werden und die Ausgänge der bool'schen Logik können direkt mit den entsprechenden Gates 9 der als Messsensor wirkenden Feldeffekttransistoren 3a, 3b, 3c verbunden werden. Unter Umständen werden damit allerdings Störeinflüsse auf die bool'sche Logik an die Steuerspannung der Gates 9 weitergegeben. Dieser Effekt kann aber minimiert werden, falls die Logiklevel "0" und "1" identisch sind mit geeigneten Versorgungspannungen U_{V}.

In Figur 4 ist ein Schaltbild einer erfindungsgemäß ausgebildeten Vorrichtung mit einem in Reihe geschalteten Referenzsensor dargestellt.

Im Unterschied zu der in Figur 1 dargestellten Ausführungsform ist bei der in Figur 4 dargestellten Ausführungsform zu den parallel geschalteten, als Messsensoren dienenden Feldeffekttransistoren 3a, 3b, 3c ein Referenzsensor 35 in Reihe geschaltet. Der Referenzsensor 35 dient zur Kompensation von Offset-Effekten. Üblicherweise ist der Referenzsensor derart ausgebildet, dass dieser nicht von den zu detektierenden Substanzen beeinflusst wird. Ansonsten ist der Aufbau des Referenzsensors 35 jedoch identisch zu dem der als Messsensoren wirkenden Feldeffekttransistoren 3a, 3b, 3c. Das heißt, dass in der hier dargestellten Ausführungsform der Referenzsensor 35 ebenfalls ein Feldeffekttransistor ist. Damit der Referenzsensor 35 gegenüber den zu detektierenden Substanzen unempfindlich ist, wird im Allgemeinen die Gate-Elektrode 9 passiviert.

In der in Figur 4 dargestellten Ausführungsform ist der Referenzsensor 35 in Reihe zu den parallel geschalteten, als Messsensoren dienenden Feldeffekttransistoren 3a, 3b und 3c geschaltet. Die Gate-Elektrode 9 des als Referenzsensor dienenden Feldeffekttransistors 35 ist auf den Arbeitspunkt festgelegt. In der hier dargestellten Ausführungsform wird der Arbeitspunkt bei 0 V gewählt. Hierdurch sind der Arbeitspunkt und die Source-Spannung U_{S} identisch, und am Referenzsensor 35 können Gate-Elektrode 9 und Source-Elektrode 5 kurzgeschlossen werden. Als Strom durch die Signalleitung 5 wird somit nur noch der Differenzstrom zwischen dem Referenzsensor 35 und dem als Messsensor wirkenden Feldeffekttransistor 3a, 3b, 3c gemessen, an dessen Gate-Elektrode 9 die Gatespannung im Arbeitspunkt U_{A} angelegt ist. Um eine sinnvolle Kompensation zu ermöglichen, müssen die als Messsensor wirkenden Feldeffekttransistoren 3a, 3b, 3c und der Referenzsensor 35 idealerweise ein gleiches oder zumindest ähnliches Verhalten im Hinblick auf mögliche Störeinflüsse, wie zum Beispiel Temperaturschwankungen, aufweisen. Wenn die Source-Spannung U_{S} und Referenz-Spannung U_{Ref} symmetrisch zur Signalspannung U_{Sig} gewählt werden, sind die Offset-Ströme für den Referenzsensor 35 und den als Messsensor dienenden Feldeffekttransistor 3a, 3b, 3c, an dessen Gate-Elektrode 9 die Gatespannung im Arbeitspunkt U_{A} angelegt wird, gleich, und das gemessene Stromsignal I zeigt nur noch die Änderung aufgrund der Beeinflussung des messenden Feldeffekttransistors 3a, 3b, 3c durch die zu detektierende Substanz.

Wenn ein Arbeitspunkt unterschiedlich von einer Source-Gate-Spannung von 0 V gewählt wird, muss auch der Referenzsensor 35 auf einen entsprechenden Arbeitspunkt festgelegt werden. Weiterhin ist es auch denkbar, eine individuelle Abstimmung, d.h. einen Nullabgleich, zwischen den als Messsensoren dienenden Feldeffekttransistoren 3a, 3b, 3c und dem Referenzsensor 35 durchzuführen, indem die Arbeitspunkte für die als Messsensoren dienenden Feldeffekttransistoren 3a, 3b, 3c nicht identisch gewählt werden und so Abweichungen zwischen den als Messsensoren dienenden Feldeffekttransistoren 3a, 3b, 3c korrigiert werden.

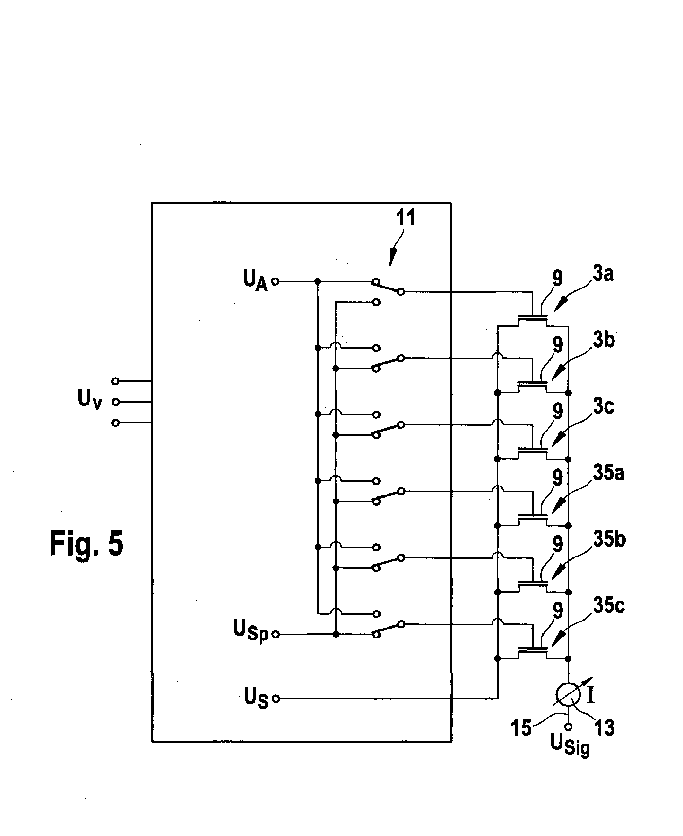

In Figur 5 ist ein Schaltbild einer erfindungsgemäßen Vorrichtung mit Referenzsensoren in einer zweiten Ausführungsform dargestellt.

Im Unterschied zu der in Figur 4 dargestellten Ausführungsform sind bei der in Figur 5 dargestellten Ausführungsform mehrere Referenzsensoren 35a, 35b und 35c zu den als Messsensor dienenden Feldeffekttransistoren 3a, 3b, 3c parallel geschaltet. Alternativ zu der hier dargestellten Ausführungsform, bei der zu jedem als Messsensor dienenden Feldeffekttransistor 3a, 3b, 3c ein Referenzsensor 35a, 35b, 35c vorgesehen ist, ist es auch möglich, für mehrere als Messsensor dienende Feldeffekttransistoren 3a, 3b, 3c jeweils nur einen Referenzsensor vorzusehen. So ist es zum Beispiel auch möglich, dass zu sämtlichen als Messsensor dienenden Feldeffekttransistoren 3a, 3b, 3c nur ein einziger Referenzsensor 35 parallel geschaltet wird. Vorteil der in Figur 5 dargestellten Ausführungsform, bei der zu jedem als Messsensor dienenden Feldeffekttransistor 3a, 3b, 3c ein Referenzsensor 35a, 35b, 35c parallel geschaltet wird, ist, dass Feldeffekttransistoren mit unterschiedlicher elektrischer Charakteristik verwendet werden können, wie sie zum Beispiel durch unterschiedlichen Aufbau oder Dimensionen von Gate-Elektrode 9 und Kanal bedingt ist. Diese können dann in einer Anordnung integriert werden. Bei der in Figur 5 dargestellten Ausführungsform werden die als Messsensor dienenden Feldeffekttransistoren 3a, 3b, 3c und die Referenzsensoren 35a, 35b, 35c sequenziell ausgelesen. Die Signale werden einer Auswerteschaltung zur Verfügung gestellt, und in der Auswerteschaltung werden die Signale der Referenzsensoren 35a, 35b, 35c zur Kompensation von Störungen, zum Beispiel Temperaturschwankungen, genutzt.

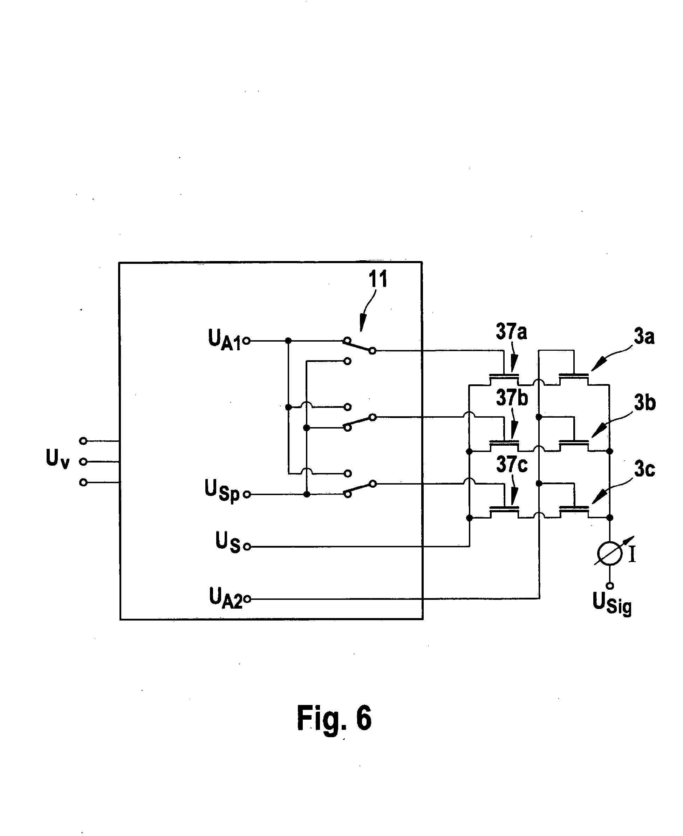

In Figur 6 ist ein Schaltbild einer erfindungsgemäßen Vorrichtung mit Feldeffekttransistoren als Mittel zum Schalten dargestellt.

Im Unterschied zu den vorstehend dargestellten Ausführungsformen ist bei der in Figur 6 dargestellten Ausführungsform jedem als Messsensor dienenden Feldeffekttransistor 3a, 3b, 3c jeweils ein Schalt-Feldeffekttransistor 37a, 37b, 37c vorgeschaltet. Hierbei ist jeweils die Drain-Elektrode eines der Schalt-Feldeffekttransistoren 37a, 37b, 37c mit der Source-Elektrode eines der als Messsensor wirkenden Feldeffekttransistoren 3a, 3b, 3c verbunden. Das heißt, dass jeweils einer der als Messsensor wirkenden Feldeffekttransistoren 3a, 3b, 3c mit einem Schalt-Feldeffekttransistor 37a, 37b, 37c in Reihe geschaltet ist. Die Gate-Elektroden der Schalt-Feldeffekttransistoren 37a, 37b, 37c sind jeweils mit dem Ausgang der Mittel zum Schalten 11 verbunden. An die Eingänge der Mittel zum Schalten sind einmal eine Sperrspannung R_{Sp} und zum anderen eine Gate-Spannung im Arbeitspunkt U_{A1} für den Schalt-Feldeffekttransistor 37a, 37b, 37c angelegt. An die Gate-Elektroden der als Messsensor dienenden Feldeffekttransistoren 3a, 3b, 3c ist eine Gate-Spannung im Arbeitspunkt U_{A2} für die als Messsensor dienenden Feldeffekttransistoren 3a, 3b, 3c angelegt. Durch die Mittel zum Schalten 11 wird jeweils ein Schalt-Feldeffekttransistor 37a, 37b, 37c mit der Gate-Spannung im Arbeitspunkt U_{A1} versorgt, während die anderen Schalt-Feldeffekttransistoren 37a, 37b, 37c gesperrt sind, das heißt an diese die Sperrspannung U_{Sp} angelegt ist. Durch die in Figur 6 dargestellte Anordnung wird vermieden, dass das elektro-chemische Gleichgewicht des chemisch sensitiven Gates des als Messsensor wirkenden Feldeffekttransistors 3a, 3b, 3c durch den Schaltvorgang beeinflusst wird, da an alle als Messsensoren wirkenden Feldeffekttransistoren 3a, 3b, 3c eine konstante Gate-Spannung angelegt werden kann, die durch den Schaltvorgang nicht verändert wird. Zudem erlaubt die Trennung von Schaltung und Fluid-Detektion eine Optimierung der verwendeten Feldeffekttransistoren 3a, 3b, 3c; 37a, 37b, 37c auf ihre jeweilige Aufgabe. So sind insbesondere bei den als Messsensor wirkenden Feldeffekttransistoren 3a, 3b, 3c aufgrund der elektro-chemischen Anforderungen an die GateGeometrie höhere Leckströme und ein schlechteres Abschnürverhalten zu erwarten als bei einem zur Schaltung optimierten Feldeffekttransistor 37a, 37b, 37c.

## Patentansprüche

1. Vorrichtung zur Detektierung von in einem Gasstrom enthaltenen Substanzen, mindestens zwei Feldeffekttransistoren (3a, 3b, 3c) als Messsensoren umfassend, wobei die Feldeffekttransistoren (3a, 3b, 3c) jeweils eine Gate-Elektrode (9), eine Source-Elektrode (5) und eine Drain-Elektrode (7) aufweisen, wobei die Source-Elektroden (5) und die Drain-Elektroden (7) aller als Messsensoren wirkenden Feldeffekttransistoren (3a, 3b, 3c) parallel geschaltet sind, **dadurch gekennzeichnet, dass** die Vorrichtung (1) weiterhin Mittel zum Schalten (11) umfasst, so eingerichtet dass eine Gate-Spannung im Arbeitspunkt (UA) und eine Sperrspannung (USp) an den Eingängen der Mittel zum Schalten (11) angelegt sind und die Gate-Elektrode (9) jedes Feldeffekttransistors (3a, 3b, 3c) mit einem Ausgang eines Mittels zum Schalten (11) verbunden ist, so dass an alle Feldeffekttransistoren (3a, 3b, 3c) unabhängig voneinander die Gate-Spannung im Arbeitspunkt (UA) oder die Sperrspannung (USp) angelegt werden kann,

2. Vorrichtung gemäß Anspruch 1, **dadurch gekennzeichnet, dass** die Vorrichtung (1) weiterhin eine digitale Schaltung (23) umfasst, die das Anlegen der Gate-Spannung im Arbeitspunkt (UA) oder der Sperrspannung (USp) an die Feldeffekttransistoren (3a, 3b, 3c) steuert.

3. Vorrichtung gemäß Anspruch 2, **dadurch gekennzeichnet, dass** die digitale Schaltung (23) einen Taktgenerator (25), einen sequenziellen Zähler (27) und eine bool'sche Lo-gik (29) umfasst.

4. Vorrichtung gemäß einem der Ansprüche 1 bis 3, **dadurch gekennzeichnet, dass** alle als Messsensoren wirkenden Feldeffekttransistoren (3a, 3b, 3c) an eine gemeinsame Signalleitung (15) angeschlossen sind.

5. Vorrichtung gemäß einem der Ansprüche 1 bis 4, **dadurch gekennzeichnet, dass** als Mittel zum Schalten (11) Schalt-Feldeffekttransistoren (37a, 37b, 37c) verwendet wer-den, die jeweils zu den als Messsensor wirkenden Feldeffekttransistoren (3a, 3b, 3c) in Reihe geschaltet sind.

6. Vorrichtung gemäß einem der Ansprüche 1 bis 5, **dadurch gekennzeichnet, dass** die Feldeffekttransistoren (3a, 3b, 3c) mit einem Referenzsensor (35; 35a, 35b, 35c) ver-bunden sind, um Störgrößen zu eliminieren.

7. Vorrichtung gemäß Anspruch 6, **dadurch gekennzeichnet, dass** ein Referenzsensor (35) mit allen parallel geschalteten als Messsensor wirkenden Feldeffekttransistoren (3a, 3b, 3c) in Reihe geschaltet ist.

8. Vorrichtung gemäß Anspruch 6, **dadurch gekennzeichnet, dass** ein Referenzsensor (35) zu allen parallel geschalteten als Messsensor wirkenden Feldeffekttransistoren (3a, 3b, 3c) parallel geschaltet ist.

9. Vorrichtung gemäß Anspruch 6, **dadurch gekennzeichnet, dass** zu jedem als Messsen-sor wirkenden Feldeffekttransistor (3a, 3b, 3c) ein Referenzsensor (35a, 35b, 35c) pa-rallel geschaltet ist.

10. Vorrichtung gemäß einem der Ansprüche 6 bis 9, **dadurch gekennzeichnet, dass** der Referenzsensor (35; 35a, 35b, 35c) ein Feldeffekttransistor ist, der gegenüber den zu detektierenden Substanzen unempfindlich ist.

11. Verfahren zur Detektierung von Substanzen in einem Gasstrom, bei dem mehrere als Messsensoren wirkende Feldeffekttransistoren (3a, 3b, 3c) parallel geschaltet sind und jeweils an einen Feldeffekttransistor (3a, 3b, 3c) eine Gate-Spannung im Arbeitspunkt (UA) und an alle anderen Feldeffekttransistoren (3a, 3b, 3c) eine Sperrspannung (USp) angelegt ist, so dass jeweils nur ein Feldeffekttransistor (3a, 3b, 3c) ein Messsignal liefert.

12. Verfahren gemäß Anspruch 11, **dadurch gekennzeichnet, dass** die Gate-Spannung im Arbeitspunkt (UA) sequenziell an einen Feldeffekttransistor (3a, 3b, 3c) nach dem an-deren angelegt wird.

13. Verfahren gemäß Anspruch 11 oder 12, **dadurch gekennzeichnet, dass** zur Eliminie-rung von Störgrößen ein Messwert aus dem Messsignal und einem Referenzsignal eines Referenzsensors (35; 35a, 35b, 35c) gebildet wird.

## Claims

1. Device for detecting substances contained in a gas flow, comprising at least two field effect transistors (3a, 3b, 3c) as measuring sensors, wherein the field effect transistors (3a, 3b, 3c) each have a gate electrode (9), a source electrode (5) and a drain electrode (7), wherein the source electrodes (5) and the drain electrodes (7) of all the field effect transistors (3a, 3b, 3c) acting as measuring sensors are connected in parallel, **characterized in that** the device (1) furthermore comprises means for switching (11), designed such that a gate voltage at the operating point (UA) and a reverse voltage (USp) are applied at the inputs of the means for switching (11) and the gate electrode (9) of each field effect transistor (3a, 3b, 3c) is connected to an output of a means for switching (11), such that the gate voltage at the operating point (UA) or the reverse voltage (USp) can be applied to all the field effect transistors (3a, 3b, 3c) independently of one another.

2. Device according to Claim 1, **characterized in that** the device (1) furthermore comprises a digital circuit (23), which controls the application of the gate voltage at the operating point (UA) or of the reverse voltage (USp) to the field effect transistors (3a, 3b, 3c).

3. Device according to Claim 2, **characterized in that** the digital circuit (23) comprises a clock generator (25), a sequential counter (27) and a Boolean logic (29).

4. Device according to any of Claims 1 to 3, **characterized in that** all the field effect transistors (3a, 3b, 3c) acting as measuring sensors are connected to a common signal line (15) .

5. Device according to any of Claims 1 to 4, **characterized in that** switching field effect transistors (37a, 37b, 37c) are used as means for switching (11), said switching field effect transistors respectively being connected in series with the field effect transistors (3a, 3b, 3c) acting as measuring sensor.

6. Device according to any of Claims 1 to 5, **characterized in that** the field effect transistors (3a, 3b, 3c) are connected to a reference sensor (35; 35a, 35b, 35c) in order to eliminate disturbance variables.

7. Device according to Claim 6, **characterized in that** a reference sensor (35) is connected in series with all the parallel-connected field effect transistors (3a, 3b, 3c) acting as measuring sensor.

8. Device according to Claim 6, **characterized in that** a reference sensor (35) is connected in parallel with all the parallel-connected field effect transistors (3a, 3b, 3c) acting as measuring sensor.

9. Device according to Claim 6, **characterized in that** a reference sensor (35a, 35b, 35c) is connected in parallel with each field effect transistor (3a, 3b, 3c) acting as measuring sensor.

10. Device according to any of Claims 6 to 9, **characterized in that** the reference sensor (35; 35a, 35b, 35c) is a field effect transistor that is insensitive to the substances to be detected.

11. Method for detecting substances in a gas flow, wherein a plurality of field effect transistors (3a, 3b, 3c) acting as measuring sensors are connected in parallel and a gate voltage at the operating point (UA) is applied in each case to one field effect transistor (3a, 3b, 3c) and a reverse voltage (USp) is applied to all the other field effect transistors (3a, 3b, 3c), such that in each case only one field effect transistor (3a, 3b, 3c) supplies a measurement signal.

12. Method according to Claim 11, **characterized in that** the gate voltage at the operating point (UA) is applied sequentially to one field effect transistor (3a, 3b, 3c) after the other.

13. Method according to Claim 11 or 12, **characterized in that**, in order to eliminate disturbance variables, a measurement value is formed from the measurement signal and a reference signal of a reference sensor (35; 35a, 35b, 35c).

## Revendications

1. Dispositif de détection de substances que contient un écoulement de gaz,
comprenant comme capteur de mesure au moins deux transistors (3a, 3b, 3c) à effet de champ,
les transistors (3a, 3b, 3c) à effet de champ présentant tous une électrode de grille (9), une électrode de source (5) et une électrode de drain (7),
les électrodes de source (5) et les électrodes de drain (7) de tous les transistors (3a, 3b, 3c) à effet de champ fonctionnant comme capteurs de mesure étant raccordées en parallèle,
**caractérisé en ce que**
le dispositif (1) comporte en outre des moyens de commutation (11) conçus de telle sorte qu'une tension de grille soit appliquée au point de travail (UA) et une tension de blocage (USp) aux entrées des moyens de commutation (11) et que l'électrode de grille (9) de chaque transistor (3a, 3b, 3c) à effet de champ soit raccordée à une sortie d'un moyen de commutation (11) de telle sorte que de manière indépendante pour tous les transistors (3a, 3b, 3c) à effet de champ, la tension de grille puisse être appliquée au point de travail (UA) ou la tension de blocage (USp) puisse être appliquée.

2. Dispositif selon la revendication 1, **caractérisé en ce que** le dispositif (1) comporte en outre un circuit numérique (23) qui commande l'application de la tension de grille au point de travail (UA) ou de la tension de blocage (USp) sur les transistors (3a, 3b, 3c) à effet de champ.

3. Dispositif selon la revendication 2, **caractérisé en ce que** le circuit numérique (23) comporte un générateur de cadence d'horloge (25), un compteur séquentiel (27) et une logique de Boole (29).

4. Dispositif selon l'une des revendications 1 à 3, **caractérisé en ce que** tous les transistors (3a, 3b, 3c) à effet de champ qui fonctionnent comme capteurs de mesure sont raccordés à un conducteur de signaux (15) commun .

5. Dispositif selon l'une des revendications 1 à 4, **caractérisé en ce que** des transistors (37a, 37b, 37c) à effet de champ de commutation sont utilisés comme moyens de commutation (11) et sont tous raccordés en série sur les transistors (3a, 3b, 3c) à effet de champ qui fonctionnent comme capteurs de mesure.

6. Dispositif selon l'une des revendications 1 à 5, **caractérisé en ce que** les transistors (3a, 3b, 3c) à effet de champ sont raccordés à un capteur de référence (35; 35a, 35b, 35c) pour éliminer les grandeurs parasites.

7. Dispositif selon la revendication 6, **caractérisé en ce qu'**un capteur de référence (35) est raccordé en série sur tous les transistors (3a, 3b, 3c) à effet de champ raccordés en parallèle et travaillant comme capteurs de mesure.

8. Dispositif selon la revendication 6, **caractérisé en ce qu'**un capteur de référence (35) est raccordé en parallèle à tous les transistors (3a, 3b, 3c) à effet de champ raccordés en parallèle et fonctionnant comme capteurs de mesure.

9. Dispositif selon la revendication 6, **caractérisé en ce qu'**un capteur de référence (35a, 35b, 35c) est raccordé en parallèle à chaque transistor (3a, 3b, 3c) à effet de champ travaillant comme capteur de mesure.

10. Dispositif selon l'une des revendications 6 à 9, **caractérisé en ce que** le capteur de référence (35; 35a, 35b, 35c) est un transistor à effet de champ insensible aux substances à détecter.

11. Procédé de détection de substances dans un écoulement de gaz, dans lequel plusieurs transistors (3a, 3b, 3c) à effet de champ travaillant comme capteurs de mesure sont raccordés en parallèle, une tension de grille étant appliquée au point de travail (UA) chaque fois à un transistor (3a, 3b, 3c) à effet de champ et une tension de blocage (USp) étant appliquée à tous les autres transistors (3a, 3b, 3c) à effet de champ, de sorte qu'un seul transistor (3a, 3b, 3c) à effet de champ délivre un signal de mesure..

12. Procédé selon la revendication 11, **caractérisé en ce que** la tension de grille est appliquée au point de travail (UA) séquentiellement sur un transistor (3a, 3b, 3c) à effet de champ après l'autre.

13. Procédé selon les revendications 11 ou 12, **caractérisé en ce que** pour éliminer les grandeurs parasites, une valeur de mesure est formée à partir du signal de mesure et d'un signal de référence d'un capteur de référence (35; 35a, 35b, 35c).
